# EUROPEAN PATENT APPLICATION

(11) **EP 3 516 967 A1**
(43) Date of publication of application: **31.07.2019**
(21) Application number: 17853211.5
(22) Date of filing: 25.09.2017
(51) Int. Cl.: A23L 5/43, A23L 29/10, A61K 8/34, A61K 8/73, A61K 9/10, A61K 31/12, A61K 47/10, A61K 47/36, C08B 11/08, C08B 15/10, C08B 31/04, C08B 37/00, C09B 61/00, C09B 67/20, C09B 67/46

(54) **CURCUMIN COMPOSITION**

(30) Priority: 26.09.2016 JP 2016186524
(71) Applicant: San-Ei Gen F.F.I., INC., Toyonaka-shi, Osaka 561-8588 (JP)
(72) Inventor: MIYAMOTO, Kanako, Toyonaka-shi Osaka 561-8588 (JP); SAKATA, Makoto, Toyonaka-shi Osaka 561-8588 (JP)
(74) Representative: Hoffmann Eitle
(86) International application number: PCT/JP2017/034574
(87) International publication number: WO 2018/056444

(57) **Abstract**

The present invention provides a curcumin-containing composition in which aggregation upon storage is suppressed or prevented.

The object can be attained by a composition comprising curcumin, gum arabic, modified starch, and water, and having a median diameter of 1 µm or less.

## Description

### Technical Field

The present invention relates to a curcumin composition and the like.

### Background Art

Turmeric is a food material that has been widely used as a natural food additive or a spice. Various foods and beverages containing turmeric are available in the marketplace. On the other hand, it has recently been reported that turmeric has an antioxidant effect, an antiinflammatory effect, and an anticancer effect. Further, clinical study of turmeric regarding a cardiac failure prevention effect and a medicinal effect against cancers and Alzheimer have been carried out, and turmeric has thus been attracting attention also as a functional natural material. Turmeric (Turmeric, Curcuma longaL.) comprises curcumin as a yellow main component, and research on its bioactivity has also been conducted.

Since curcumin is a hydrophobic substance, to prepare an aqueous curcumin preparation, it is necessary to suspend curcumin in water using a dispersant.

For example, Patent Document 1 discloses, as a method for producing a water dispersible pigment composition containing a water-insoluble and/or hydrophobic pigment dispersion, "a method for preparing a water dispersible pigment composition containing more than 10 wt% of water, the method comprising preparing a water-insoluble and/or hydrophobic natural pigment dispersion by obtaining a dispersion containing a pigment in the form of a material having an average size of 10 µm or less by pulverizing a water-insoluble and/or hydrophobic natural pigment in an aqueous phase containing a hydrocolloid in an amount of at least 1 wt% based on the pigment amount in the absence of an emulsifier, wherein the composition contains more than 10 wt% of water." Patent Document 1 discloses gum arabic as an example of the hydrocolloid, and also discloses curcumin as a hydrophobic natural pigment.

### Citation List

### Patent Documents

Patent Document 1: Japanese Patent No. 3176934

### Summary of Invention

### Technical Problem

However, the research by the inventors of the present invention revealed that:
(1) A curcumin aqueous preparation can be prepared by pulverizing curcumin in water and in the presence of gum arabic, thereby dispersing curcumin in water; however, after storing the preparation for a long period of time, aggregation occurs; and
(2) In particular, when polyhydric alcohol, which is commonly used as a preservative, is added upon the preparation of the curcumin aqueous preparation, solid aggregation occurs during the production step, and the preparation cannot be produced.

Therefore, an object of the present invention is to suppress or prevent the aggregation of a curcumin aqueous preparation, and also to suppress or prevent aggregation during the production of a curcumin aqueous preparation.

Another object of the present invention is to provide a curcumin-containing composition in which aggregation upon storage is suppressed or prevented.

### Solution to Problem

As a result of extensive research, the present
inventors found that the above problems can be solved by a composition containing curcumin, gum arabic, modified starch, and water. Thus, the present invention has been completed.

The present invention encompasses the following aspects.
Item 1.
   A composition comprising curcumin, gum arabic, modified starch, and water, and having a median diameter of 1 µm or less.
Item 2.
   The composition according to Item 1, wherein the composition is a liquid composition.
Item 3.
   The composition according to Item 1 or 2, wherein the composition contains curcumin as a turmeric pigment.
Item 4.
   The composition according to any one of Items 1 to 3, wherein the amount of curcumin is in a range of 0.5 to 30 mass%.
Item 5.
   The composition according to any one of Items 1 to 4, wherein the gum arabic is modified gum arabic.
Item 6.
   The composition according to any one of Items 1 to 5, wherein the amount of gum arabic is in a range of 10 to 250 parts by mass, per 100 parts by mass of curcumin.
Item 7.
   The composition according to any one of Items 1 to 6, wherein the amount of modified starch is in a range of 0.5 to 100 parts by mass, per 100 parts by mass of curcumin.
Item 8.
   The composition according to any one of Items 1 to 7, wherein the amount of modified starch is in a range of 0.5 to 300 parts by mass, per 100 parts by mass of gum arabic.
Item 9.
   The composition according to any one of Items 1 to 8, wherein the composition further contains polyhydric alcohol.
Item 10.
   The composition according to Item 9, wherein the polyhydric alcohol is glycerol.
Item 11.
   The composition according to Item 9 or 10, wherein the amount of polyhydric alcohol is in a range of 10 to 1000 parts by mass, per 100 parts by mass of curcumin.
Item 12.
   A method for producing the composition according to Items 1 to 11, comprising mixing curcumin, gum arabic, and water in the presence of modified starch.
Item 13.
   The method for producing the composition according to Item 12, the method comprising mixing curcumin, gum arabic, water, and polyhydric alcohol in the presence of modified starch.
Item 14.
   The method according to Item 12 or 13, the method comprising pulverizing the mixture obtained by the mixing.
Item 15.
   The composition according to any one of Items 1 to 11, wherein the composition is a pigment preparation.
Item 16.
   A food product colored with the composition according to Item 15.
Item 17.
   A dispersant for curcumin, comprising gum arabic and modified starch.
Item 18.
   The dispersant according to Item 17, wherein the dispersant is an aggregation inhibitor for curcumin.

### Advantageous Effects of Invention

The present invention suppresses or prevents the aggregation of a curcumin aqueous preparation, and also suppresses or prevents aggregation during the production of a curcumin aqueous preparation.

The present invention further provides a curcumin-containing composition in which aggregation upon storage is suppressed or prevented.

### Brief Description of Drawings

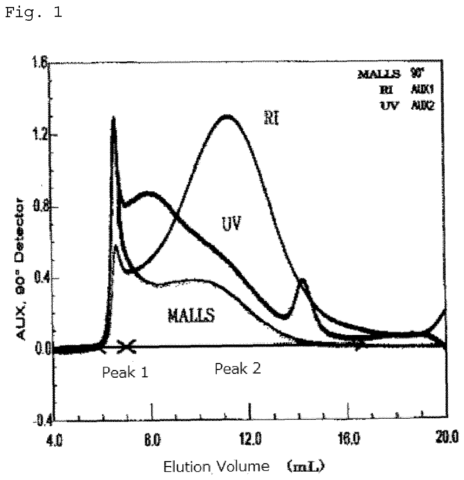

Fig. 1: A chromatogram showing results of analysis of unmodified gum arabic (A. senegal) by the GPC-MALLS method.

### Description of Embodiments

### [1] Terms

The symbols and the abbreviations in this specification are to be interpreted as having general meanings in the related technical field to which the present invention pertains, according to the context of this specification, unless otherwise specified.

In the present specification, the term "comprise" or "contain" is used with the intention of including the terms "consist essentially of" and "consist of."

The steps, treatments, or operations in this specification can be performed at room temperature, unless otherwise specified.

In this specification, room temperature refers to a temperature in a range of 10 to 40°C. In this specification, a median diameter is based on volume.

### [2] Curcumin Composition

The composition of the present invention comprises curcumin, gum arabic, modified starch, and water. Specifically, the composition of the present invention is a curcumin-containing composition.

The composition of the present invention is preferably a liquid composition. Specifically, the composition of the present invention is preferably an aqueous preparation (or, an aqueous suspension preparation).

In this specification, the term "liquid" refers to a state that is not a solid or a gas, and that has fluidity.

In this specification, the term "liquid" includes a semisolid state.

In the liquid composition of the present invention (or the composition of the present invention as an aqueous suspension preparation), curcumin is dispersed in water.

The composition of the present invention preferably has fluidity.

The presence of fluidity of the composition of the present invention can be determined by using a tube inversion method, which is commonly used to determine gelation.

More specifically, when 25 g of the composition of the present invention is placed in a screw tube having an inner diameter of 3 cm and a depth of 5.5 cm, and adjusted in temperature to 50°C, and then the screw tube is inverted at 120°, 50 mass% or more of the composition goes outside the screw tube within 5 seconds.

### [3] Curcumin

The curcumin used in the present invention is preferably in a form contained in a turmeric pigment or a turmeric extract.

The curcumin used in the present invention is, for example, curcumin obtainable from the rhizome of Curcuma longa Linne.

Preferably, the curcumin used in the present invention is obtained by subjecting dried turmeric rhizome (turmeric powder) to extraction using warmed ethanol, using a heated oil or fat or propylene glycol, or using hexane or acetone at room temperature or heated hexane or acetone.

The curcumin used in the present invention is preferably a solid (crystal, amorphia, or a combination thereof), and more preferably a crystal.

Such curcumin can be obtained, for example, by extracting turmeric powder using hexane and acetone, subjecting the resulting extract to filtration, and volatilizing the solvent to dry it.

Alternatively, synthesized curcumin products may be used.

The curcumin used in the present invention may not be pure curcumin, and may be curcumin having a purity of, for example, 70 mass% or more, 80 mass% or more, 90 mass% or more, 95 mass%, or 97 mass%.

Conveniently, commercially available turmeric pigments (curcumin powder: crystalline) can be used. Such curcumin powders are commercially available from San-Ei Gen F.F.I., Inc., etc.

Preferable examples of the turmeric pigment include turmeric pigments containing at least 80 mass% of curcumin.

The curcumin contained in the composition of the present invention has a median diameter of 1 µm or less, preferably 0.9 µm or less, more preferably 0.7 µm or less, and further preferably 0.5 µm or less.

The lower limit of the median diameter of the curcumin used in the present invention is not particularly limited; however, the lower limit may be, for example, 0.01 µm, 0.05 µm, or 0.1 µm.

The median diameter of the turmeric pigment in the turmeric pigment liquid composition of the present invention, in other words, the median diameter of the turmeric pigment in a gum ghatti solution, is measurable using a (wet) laser diffraction particle size distribution analyzer Microtrac MT-3000II (product name, Microtrac Corporation) (measurement conditions: refractive index = 1.81, measurement range = 0.021 to 2000 µm, particle size distribution: volume-based).

The amount of curcumin contained in the composition of the present invention is preferably 0.5 to 30 mass%, more preferably 1 to 25 mass%, further preferably 1.5 to 20 mass%, further more preferably 2 to 18 mass%, particularly preferably 2 to 16 mass%, more particularly preferably 2 to 14 mass%, and further particularly preferably 3 to 12 mass%.

When the composition of the present invention contains a turmeric pigment as a curcumin source, the amount of the turmeric pigment is preferably 0.5 to 30 mass%, more preferably 1 to 25 mass%, further preferably 2 to 20 mass%, further more preferably 3 to 18 mass%, and particularly preferably 3 to 13 mass%.

### Gum Arabic

Various kinds of gum arabic may be used in the present invention. In this specification, the term "gum arabic" encompasses "natural gum arabic" or "unmodified gum arabic", and "modified gum arabic."

Gum arabic is a natural exudate obtained from the trunks and branches of a plant of the genus Acacia (especially, Acacia senegal and Acacia seyal) of the Leguminasae family. Gum arabic is highly soluble in water and its aqueous solution provides high emulsifiability, emulsion stability, encapsulation ability, adhesiveness, protective colloid property and film-forming ability even at a relatively low concentration, so it has been widely used as an emulsifier, thickener, stabilizer, binder, and coating agent.

One embodiment of the present invention uses natural gum arabic (unmodified gum arabic) as gum arabic. Examples of the natural gum arabic (unmodified gum arabic) include unmodified gum arabic listed below as the raw materials for the production of modified gum arabic.

A preferred embodiment of the present invention uses modified gum arabic as gum arabic.

Although modified gum arabic is a known substance disclosed in the internationally-published patent application WO2004/089991, and can be understood from the disclosure thereof, modified gum arabic is explained below.

The modified gum arabic usable in the present invention may originate from, for example, the species Acacia senegal or Acacia seyal obtained by treating natural gum arabic belonging to the species Acacia senegal or Acacia seyal.

The natural gum arabic belonging to these species have different molecular weights, different protein distributions and different molecular properties, due to their structural differences.

For example, the natural gum arabic belonging to A.senegal is laevorotatory and has the specific optical rotation of approximately -30 degrees. In contrast, the natural gum arabic belonging to A.seyal is dextrorotatory and has the specific optical rotation of approximately +50 degrees. Additionally, compared with the gum arabic belonging to A.senegal, it is known that gum arabic belonging to A.seyal has a lower protein content (nitrogen content), a lower viscosity when made into an aqueous solution, and a different sugar composition.

### (1) Modified gum arabic originated from A. senegal

(1-1) The modified gum arabic usable in the present invention may be, for example, a water-soluble modified gum arabic having a weight average molecular weight of not less than 9×10⁵ (0.9 million), preferably not less than 15.0×10⁵ (1.5 million), further preferably not less than 20.0×10⁵(2 million) obtained by, for example, heating gum arabic belonging to Acacia senegal.

The weight average molecular weight is determined by the use of a gel filtration chromatography having three detectors, i.e., a light scattering (MALLS) detector, a refractive index (RI) detector and an ultraviolet (UV) detector. In this specification, a technique of such gel filtration chromatography is referred to as "GPC-MALLS." GPC-MALLS operates such that the molecular weight is measured by a MALLS detector, the mass (composition ratio) of each component is measured by an RI detector, and the protein content is measured by a UV detector. Therefore, it is possible to obtain the molecular weights and the compositions of the components to be analyzed without reference to the standard gum arabic having a known molecular weight. Detailed principals and characteristics of the GPC-MALLS can be found in a Non-Patent Document: Idris, O.H.M., Williams, P.A. Phillips, G.O.; Food Hydrocolloids, 12, (1998) pp. 375-388."

Measurement conditions for GPC-MALLS employed in the present invention are as below:

| | |
|---|---|
| • Column | : Superose (6HR) 10/30 (Pharmacia Biotech, Sweden) |
| • Flow rate | : 0.5 mL/min |
| • Eluant | : 0.2 M NaCl |
| • Preparation of a sample | : A sample to be analyzed was diluted with the eluant (0.2 M NaCl). |
| • Sample concentration | : 0.4% (W/V) |
| • Injection volume of sample solution | : 100 µL |
| • dn/dc | : 0.141 |
| • Temperature | : Room temperature |
| • Detector | : 1) MALLS (multi angle laser light scattering) detector: DAWN DSP (manufactured by Wyatt Technology Inc., USA) |
| | 2) RI (Refractive Index) detector |
| | 3) UV detector (absorption at 214 nm) |

By processing the data obtained by the GPC-MALLS conducted under the above-described conditions using ASTRA Version 4.5 (Wyatt Technology) software, each parameter of the components of the gum arabic, such as the weight average molecular weight, recovery rate (% mass), polydispersity value (P) and root mean square radius of gyration (Rg) can be obtained. When the data is processed regarding all peaks on the chromatogram obtained using an RI detector as one peak, the obtained molecular weight is identified as the weight average molecular weight (M_{wt}) (more specifically, "M_{wt} processed as one peak"). When the point where the RI chart begins to rise from the baseline of the chromatogram is defined as the "starting point," and the point where the RI chart falls and intersects the baseline is defined as the "ending point," the aforementioned "one peak" on the chromatogram means the region from the starting point to the ending point.

The weight average molecular weight of the modified gum arabic usable in the present invention is preferably not less than 9.0×10⁵ (0.9 million), more preferably not less than 12.0×10⁵ (1.2 million), further preferably not less than 15.0×10⁵ (1.5 million), further more preferably not less than 20.0×10⁵ (2 million). There is no specific upper limit to the weight average molecular weight as long as the modified gum arabic is soluble in water; however, the molecular weight is not more than 70.0×10⁵ (7 million), preferably not more than 60.0×10⁵ (6 million), more preferably not more than 50.0×10⁵ (5 million), further preferably not more than 40.0×10⁵ (4 million).

Furthermore, the modified gum arabic usable in the present invention preferably has the above-mentioned weight average molecular weight and is water-soluble. "Water-soluble" herein means a property such that a sample is almost completely or essentially completely dissolved in an excess of water. "Water" herein means any type of water, e.g., pure water, ion-exchanged water or ion-containing water. The water temperature may be any suitable temperature as long as the gum arabic is soluble. More specifically, by appropriately setting the type and the temperature of water, any modified gum arabic dissolvable in water may suitably be used.

Once gum arabic becomes hydrogelatinous, it cannot be dissolved in water even if a large amount of water is added or by heating; therefore the term "water-soluble" regarding gum arabic used in the present specification is used to distinguish the modified gum arabic usable in the present invention from hydrogelatinous gum arabic, which is insoluble in water. In other words, the modified gum arabic used in the present invention does not include modified polymeric gum arabic that is insoluble in water, such as hydrogels, etc.

Moreover, it is preferable that the modified gum arabic usable in the present invention have the above-mentioned weight average molecular weight, be water-soluble, and be the same as or similar to unmodified gum arabic in terms of immunological reactivity. The phrase "the same as or similar to unmodified gum arabic in terms of immunological reactivity" means that the difference between the degree of immunological inhibition of the modified gum arabic and that of gum arabic belonging to Acacia Senegal is within ±10%, as measured by indirect competitive ELISA using a quantifiable antibody for gum arabic, for example "SYCC7" [Thurston, M.I, et al., Detection of gum from Acacia seyal and species of combretum in mixtures with A. senegal using monoclonal antibodies, Food & Agric. Immunol., 10: 237-241(1998), Thurston, M.I., et al., Effect of heat and pH on carbohydrate epitopes from Acacia senegal by specific monoclonal antibodies, Food & Agric. Immunol., 11: 145-153 (1999)].

The form of the modified gum arabic usable in the present invention is not limited and it can take any form including blocks, beads, coarse pulverizates, granules, pellets and powders.

The modified gum arabic usable in the present invention can be prepared by heating gum arabic belonging to Acacia senegal as a raw material using a thermostat or a heater, such as an oven, at 110°C for at least 10 hours.

The unmodified gum arabic (A.senegal) used herein as a raw material is a natural exudate obtained from the trunks and branches of Acacia senegal (A.senegal) of the genus Acacia, family Leguminasae, or any other tree belonging to the same genus. The unmodified gum arabic may also be unmodified gum arabic that has been subjected to a treatment, such as purification, desalting, pulverization, or spray drying.

The unmodified gum arabic (A.senegal) is produced in countries of North and West Africa across Ethiopia to Senegal (Ethiopia, Sudan, Senegal, Nigeria, Niger, and Ghana), countries of East Africa such as Kenya and Uganda, the Sahara region of Africa and the basins of the tributaries of the Nile. The unmodified gum arabic (A.senegal) produced in any of the above areas can be used in the present invention regardless of its origin.

Further, unmodified gum arabic (A.senegal) is not particularly restricted in its water content. For commercially available unmodified gum arabic (A.senegal), reduction in water content when dried by heating at 105°C for 6 hours (loss-on-drying) is generally not more than 40 mass%, preferably not more than 30 mass%, and particularly preferably not more than 20 mass%. In the present invention, unmodified gum arabic (A.senegal) having such water contents (loss-on-drying) can be used without limitation.

Unmodified gum arabic (A.senegal) can usually be procured in the forms of blocks, beads, coarse pulverizates, granules, pellets, and powders (including spray dried powder). However, in the present invention, unmodified gum arabic (A.senegal) of any form can be used without limitation as a gum arabic raw material to be processed. For example, the unmodified gum arabic may be spray dried powder having a median diameter of about several tens to several hundreds of µm. The upper limit of the median diameter is not particularly limited; however, the median diameter is preferably 100 mm or less in view of modification efficiency. The median diameter is preferably in a range of 1 to 100 mm, more preferably 2 to 50 mm.

Examples of the methods for heating unmodified gum arabic (A.senegal) include a method of heating unmodified gum arabic at 110°C for at least 10 hours using an oven (thermostat), as mentioned above. A preferable heat treatment is performed at 110°C for at least 15 hours, more preferably for at least 24 hours, and still more preferably for at least 48 hours. Although it depends on the type of unmodified gum arabic (A.senegal) to be treated, the upper limit of the duration of heating when heated at 110°C may be about 72 hours. As long as it is possible to obtain modified gum arabic that has the molecular weight specified in the present invention and that is soluble in water, the heating method is not limited to that with the above conditions; the heating temperature, the duration of heating, the heating means, and the heating environment conditions (e.g., relative humidity, presence/absence of a closed system) can be optionally selected. For example, the effects of the present invention achieved by the heat treatment conducted under the conditions described above can also be obtained by a method wherein heating is performed at a temperature lower than 110°C for more than 10 hours, a method wherein heating is performed at a temperature higher than 110°C for a short time (e.g., 10 hours or less), or the like. Specifically, one example of the former case may be a method wherein heating is performed at 80°C for 3 days to 1 week or longer, or the like. The same effects can be achieved in less time by using microwave radiation instead of a heating means using an oven. In addition, a heat treatment in the absence of oxygen, such as under a nitrogen substitution condition, is desirable because it can prevent discoloration of the gum arabic to be produced.

(1-2) Further, for example, the modified gum arabic usable in the present invention may be a water-soluble modified gum arabic that contains 17 mass% or more of arabinogalactan protein obtained by heating gum arabic belonging to Acacia senegal.

Arabinogalactan protein (which may, hereinafter, simply be referred to as "AGP") is one of three major components of gum arabic along with arabinogalactan (AG) and glycoprotein (GP). Unmodified gum arabic (A.senegal) generally contains 5 to 15 mass% of AGP.

The proportion of AGP in gum arabic (unmodified gum arabic and modified gum arabic) may also be determined by the GPC-MALLS described above. More specifically, when the RI Chart of a chromatogram obtained by using an RI detector is divided into two parts, i.e., Peak 1 (high molecular weight elution fraction) which traces the first eluted portion, and Peak 2 (low molecular weight elution fraction) which traces the later eluted portion, and the data are then processed with ASTRA Version 4.5 (Wyatt Technology) software, the obtained recovery ratio of Peak 1 (% Mass) corresponds to the AGP content (mass%) of the gum arabic subjected to GPC-MALLS. This is explained in detail with reference to a chromatogram (Fig. 1(A)) showing the results of analysis in which unmodified gum arabic (A.senegal) was analyzed using GPC-MALLS. In the RI chromatogram, the point where the RI chart begins to rise from the baseline of the RI chart is defined as the "starting point" and the point where the RI chart falls and intercepts the baseline is defined as the "ending point." Between the starting point and the ending point, the point where the RI value becomes minimum is defined as the boundary, with the region between the starting point and the boundary being defined as Peak 1 and the region between the boundary and the ending point being defined as Peak 2.

The proportion of AGP in the modified gum arabic usable in the present invention is not specifically restricted as long as it is not less than 17 mass%; however, the proportion is preferably not less than 20 mass%. Although the upper limit is not particularly restricted as long as the modified gum arabic is soluble in water, the upper limit is generally about 30 mass%.

The modified gum arabic usable in the present invention is characterized in that it contains AGP at the above-mentioned proportion and is soluble in water. Moreover, it is preferable that the modified gum arabic usable in the present invention contain AGP at the proportion mentioned above, be water-soluble, and be the same as or similar to unmodified gum arabic in terms of immunological reactivity. "Water-soluble" and "be the same as or similar to unmodified gum arabic in terms of immunological reactivity" herein mean the same as those stated in (1-1).

The form of the modified gum arabic usable in the present invention is not particularly limited and it can take any form, including blocks, beads, coarse pulverizates, granules, pellets and powders (including spray dried powder).

The modified gum arabic usable in the present invention may be prepared, as described above, by heating unmodified gum arabic (A.senegal) at 110°C for at least 10 hours using an oven or like thermostat or heater. Examples of unmodified gum arabic (A.senegal) to be treated include the above-listed examples. Further, the heating method may be similar to those described above.

(1-3) Further, for example, the modified gum arabic usable in the present invention may be a water-soluble modified gum arabic that contains 10 mass% or more of arabinogalactan protein (AGP) and has a weight-average molecular weight of 9.0×10⁵ (0.9 million) or more obtained by heating unmodified gum arabic belonging to Acacia senegal.

The weight average molecular weight is preferably 10.0×10⁵ (1 million) or more, more preferably 12.0×10⁵ (1.2 million) or more, further preferably 15.0×10⁵ (1.5 million) or more, particularly preferably 20.0×10⁵ (2 million) or more. Its upper limit is not particularly restricted as long as the modified gum arabic is soluble in water; however, the upper limit is preferably not more than 70.0×10⁵ (7 million), more preferably not more than 60.0×10⁵ (6 million), further preferably not more than 50.0×10⁵ (5 million), and further more preferably about not more than 40.0×10⁵ (4 million).

The proportion of AGP in the modified gum arabic is preferably not less than 15 mass%, more preferably not less than 17 mass%, and further preferably not less than 20 mass%. Its upper limit is not particularly limited as long as the modified gum arabic is soluble in water; however the upper limit is preferably about 30 mass% or less.

The modified gum arabic usable in the present invention is characterized in that it has the above-mentioned properties and is soluble in water. Moreover, it is preferable that the modified gum arabic usable in the present invention have the above-mentioned weight average molecular weight and the AGP content, be water-soluble, and be the same as or similar to unmodified gum arabic in terms of immunological reactivity.

"Water-soluble" and "be the same as or similar to unmodified gum arabic in terms of immunological reactivity" herein mean the same as those stated in (1-1).

Further, the form of the modified gum arabic usable in the present invention is not particularly limited and it can take any form, including blocks, beads, coarse pulverizates, granules, pellets and powders (including spray dried powder).

The modified gum arabic usable in the present invention may be prepared, as described above, by heating unmodified gum arabic (A.senegal) at 110°C for at least 10 hours using an oven or like thermostat or heater. Examples of unmodified gum arabic (A.senegal) to be treated include the above-listed examples. Further, the heating method may be similar to those described above.

### (2) Modified gum arabic originated from Acacia seyal

(2-1) The modified gum arabic usable in the present invention is, for example, a water-soluble modified gum arabic having a weight average molecular weight of not less than 25.0×10⁵ (2.5 million) obtained by heating gum arabic belonging to Acacia seyal.

The weight-average molecular weight of the modified gum arabic usable in the present invention is not particularly limited insofar as it falls within the above range; however, the weight-average molecular weight is more preferably not less than 26.0×10⁵ (2.6 million), further preferably not less than 30.0×10⁵ (3 million). There is no specific upper limit of the weight average molecular weight as long as the modified gum arabic is soluble in water; however, the upper limit is preferably not more than 70.0×10⁵ (7 million), more preferably not more than 60.0×10⁵ (6 million), further preferably not more than 50.0×10⁵ (5 million), and further more preferably about 40.0×10⁵ (4 million).

The modified gum arabic usable in the present invention is characterized in that it has the above-mentioned weight-average molecular weight and is soluble in water. Moreover, it is preferable that the modified gum arabic usable in the present invention have the above-mentioned weight average molecular weight, be water-soluble, and be the same as or similar to unmodified gum arabic in terms of immunological reactivity.

"Water-soluble" and "be the same as or similar to unmodified gum arabic in terms of immunological reactivity" described here have the same meanings as stated in (1-1).

The form of the modified gum arabic usable in the present invention is not limited and it can take any form, including blocks, beads, coarse pulverizates, granules, pellets and powders.

The modified gum arabic usable in the present invention can be prepared by heating gum arabic belonging to Acacia seyal as a raw material using a thermostat, such as an oven, at 110°C for at least 10 hours.

The unmodified gum arabic (A.seyal) used herein as a raw material is a natural exudate obtained from the trunks and branches of Acacia seyal (A.seyal) of the genus Acacia, family Leguminasae, or any other tree belonging to the same genus. The unmodified gum arabic may be unmodified gum arabic that has been further subjected to a treatment, such as purification, desalting, pulverization, or spray drying.

The unmodified gum arabic (A.seyal) is produced in countries of North and West Africa across Ethiopia to Senegal (Ethiopia, Sudan, Senegal, Nigeria, Niger, and Ghana), countries of East Africa such as Kenya and Uganda, the Sahara region of Africa and the basins of the tributaries of the Nile. The unmodified gum arabic (A.seyal) produced in any of the above areas can be used in the present invention regardless of its origin.

Further, unmodified gum arabic (A.seyal) is not particularly restricted in its water content. Any commercially available unmodified gum arabic (A.seyal) can be used.

Unmodified gum arabic (A.seyal) can usually be procured in the forms of blocks, beads, coarse pulverizates, granules, pellets, and powders (including spray dried powdery form). However, in the present invention, unmodified gum arabic (A.seyal) of any form can be used without limitation as a gum arabic raw material to be heated. For example, the unmodified gum arabic may be a spray-dry powder having a median diameter of about several tens to several hundreds of µm. The upper limit of the median diameter is not particularly limited; however, the median diameter is preferably 100 mm or less in view of modification efficiency. The median diameter is preferably in a range of 1 to 100 mm, and more preferably 2 to 50 mm.

Examples of the methods for heating unmodified gum arabic (A.seyal) include heating methods similar to the heating method described above in regard to unmodified gum arabic (A. senegal).

(2-2) Further, for example, the modified gum arabic usable in the present invention may be a water-soluble modified gum arabic that contains 25 mass% or more of a protein-containing high molecular weight component obtained by heating gum arabic belonging to Acacia seyal.

The proportion of the protein-containing high molecular weight component in the unmodified gum arabic belonging to Acacia seyal and the modified gum arabic thereof can also be determined by the GPC-MALLS mentioned above in a similar manner. Specifically, when the RI chart of a chromatogram obtained using an RI detector is divided into two parts, i.e., Peak 1 (high molecular weight elution fraction), which traces the first eluted portion, and Peak 2 (low molecular weight elution fraction), which traces the later eluted portion, and the data are then processed with ASTRA Version 4.5 (Wyatt Technology) software, the recovery ratio of Peak 1 (% Mass) corresponds to the proportion (weight %) of the protein-containing high molecular weight component in the gum arabic. This is explained in detail with reference to the chromatogram (Fig. 2) showing the results of analysis in which unmodified gum arabic (A.seyal) is analyzed using the GPC-MALLS. In the RI chromatogram, the point where the RI chart begins to rise from the baseline of the chromatogram is defined as the "starting point" and the point where the RI chart falls and intercepts the baseline is defined as the "ending point." Between the starting point and the ending point, the point where the RI value becomes minimum is defined as the boundary, with the region between the starting point and the boundary being defined as Peak 1 and the region between the boundary and the ending point being defined as Peak 2.

As with the Acacia senegal, the protein-containing high molecular weight component (Peak 1) is one of the major components contained in the unmodified gum arabic (A.seyal). The unmodified gum arabic (A.seyal) generally contains this component at a proportion from 10 to 24 mass%.

The content of the protein-containing high molecular weight component (Peak 1) in the modified gum arabic (A.seyal) usable in the present invention is not particularly limited insofar as it falls within the above range; however, the content is more preferably 26 mass% or more, and further preferably 30 mass% or more. Its upper limit is not particularly limited as long as the modified gum arabic is soluble in water; however the upper limit is preferably about 45 mass% or less.

The modified gum arabic (A.seyal) usable in the present invention is characterized in that it contains the protein-containing high molecular weight component (Peak 1) at the proportion mentioned above and is soluble in water. Moreover, it is preferable that the modified gum arabic usable in the present invention contain the protein-containing high molecular weight component at the aforementioned proportion, be water-soluble, and be the same as or similar to unmodified gum arabic (A.seyal) in terms of immunological reactivity. "Water-soluble" and "be the same as or similar to unmodified gum arabic (A.seyal) in terms of immunological reactivity" herein mean the same as those stated in (1-1). The form of the modified gum arabic (A.seyal) usable in the present invention is not particularly limited and it can take any form, including blocks, beads, coarse pulverizates, granules, pellets and powders (including spray dried powder).

The modified gum arabic usable in the present invention may be prepared, as described above, by heating unmodified gum arabic (A.seyal) at 110°C for at least 10 hours using an oven or like thermostat. Examples of unmodified gum arabic (A.seyal) to be used herein include the above-listed examples. Further, the heating method may be similar to those described above (see (2-1)).

(2-3) Further, for example, the modified gum arabic usable in the present invention may be a water-soluble modified gum arabic that contains 22 mass% or more of a protein-containing high molecular weight component and has a weight-average molecular weight of not less than 1.5 million obtained by heating unmodified gum arabic belonging to Acacia seyal.

The weight-average molecular weight is preferably not less than 20.0×10⁵ (2 million), more preferably not less than 25.0×10⁵ (2.5 million). The upper limit of the weight average molecular weight is not particularly restricted as long as the modified gum arabic is soluble in water but is preferably about not more than 40.0×10⁵ (4.0 million). The proportion of the protein-containing high molecular weight component (Peak 1) in the modified gum arabic is preferably not less than 25 mass%, and more preferably not less than 30 mass%. The upper limit is not particularly restricted as long as the modified gum arabic is soluble in water but is generally about not more than 45 mass%.

The modified gum arabic (A.seyal) usable in the present invention is characterized in that it has the above-mentioned properties and is soluble in water. Moreover, it is preferable that the modified gum arabic usable in the present invention have the above weight-average molecular weight and contain the protein-containing high molecular weight component at the aforementioned proportion, be water-soluble, and be the same as or similar to unmodified gum arabic (A.seyal) in terms of immunological reactivity. "Water-soluble" and "be the same as or similar to unmodified gum arabic (A.seyal) in terms of immunological reactivity" described here have the same meanings as stated in (1-1).

Further, the form of the modified gum arabic (A.seyal) usable in the present invention is not particularly limited and it can take any form, including blocks, beads, coarse pulverizates, granules, pellets and powders (including spray dried powder).

The modified gum arabic usable in the present invention may be prepared, as described above, by heating unmodified gum arabic (A.seyal) at 110°C for at least 10 hours using an oven or like thermostat. Examples of unmodified gum arabic (A.seyal) to be used herein include the above-listed examples. Further, the heating method may be similar to those described above (see (2-1)).

All of the modified gum arabic (1-1) to (1-3) usable in the present invention originated from Acacia senegal are distinguishable from unmodified gum arabic used as a raw material in terms of their superior emulsifiability. Further, all of the modified gum arabic (2-1) to (2-3) usable in the present invention originated from Acacia seyal can also be individually distinguished from the unmodified gum arabic in terms of improved emulsifiability, emulsion stability, encapsulation ability, adhesiveness, protective colloid property or film-forming ability.

It is preferable that the median diameter of the modified gum arabic (in particular, modified gum arabic originated from A. senegal) usable in the present invention is generally not greater than 1 µm (more preferably not greater than 0.8 µm, further preferably not greater than 0.7 µm, and still more preferably not greater than 0.6 µm), when an emulsion is prepared using the modified gum arabic in the manner described below and the median diameter of droplets (dispersion phase) in the emulsion is measured.

### Method for Preparing an Emulsion

Gum arabic was dissolved in water, centrifuged to remove insoluble matter and prepared into 7.5%, 10%, 15% and 20% aqueous solutions of gum arabic.

200 g of medium-chain triglyceride (octanoic/decanoic acid triglyceride O.D.O. (trade name, Nisshin Oil Mills, Ltd.)) was added to and mixed with 800 g of each of these aqueous solutions while stirring, thereby obtaining mixtures.

The mixtures were homogenized 4 times at a pressure of 44 MPa (450 kg/cm²) using a homogenizer (manufactured by APV Gaulin) and thus were emulsified, thereby preparing emulsions.

### Method for Measuring Median Diameter of Emulsion

The median diameter of the resulting emulsions was measured immediately after emulsification and after 2 days of storage at 60°C using a particle size distribution analyzer (SALD-1100 Laser Diffraction Particle Size Analyzer, manufactured by Shimadzu Corporation).

In a particularly-preferred embodiment of the present invention, the "modified gum arabic" used as gum arabic may be a water-soluble modified gum arabic obtained from gum arabic belonging to Acacia senegal, and may be such that the weight-average molecular weight (Mwt) is not less than 0.9 million or the content of the arabinogalactan protein (%Mass) is not less than 17 mass%, and that the root mean square radius of gyration (Rg) of the arabinogalactan protein is 42.3 to 138 nm.

The "weight-average molecular weight (Mwt)" used herein means a molecular weight obtained by data processing in which all of the chromatograms determined by an RI detector in the GPC-MALLS is regarded as one peak. The "content of the arabinogalactan protein (%Mass)" and the "root mean square radius of gyration (Rg) of the arabinogalactan protein" respectively mean the content of the arabinogalactan protein and the root mean square radius of gyration (Rg) of the arabinogalactan protein of Peak 1 when data processing is performed by dividing the RI chart of a chromatogram obtained using an RI detector in the GPC-MALLS into two parts, i.e., Peak 1 which traces the first eluted portion, and Peak 2 which traces the later eluted portion.

Examples of the modified gum arabic satisfying the above conditions include SUPER GUM manufactured by San-Ei Gen F.F.I., Inc. This product is a water-soluble modified gum arabic obtained from gum arabic belonging to Acacia senegal having properties such that the weight-average molecular weight (Mwt) is not less than 0.9 million (e.g., about 3 to 5 million), the content of the arabinogalactan protein (%Mass) is not less than 17 mass%, and the root mean square radius of gyration (Rg) of the arabinogalactan protein is 42.3 to 138 nm. This product is modified gum arabic prepared by heating gum arabic belonging to Acacia senegal at 110°C for at least 10 hours.

The amount of gum arabic in the composition of the present invention is preferably 10 to 250 parts by mass, more preferably 10 to 200 parts by mass, further preferably 15 to 180 parts by mass, further more preferably 15 to 160 parts by mass, particularly preferably 20 to 130 parts by mass, and more particularly preferably 40 to 110 parts by mass, per 100 parts by mass of curcumin.

Further, the amount of gum arabic in the composition of the present invention is preferably 0.5 to 20 mass%, more preferably 1 to 18 mass%, further preferably 2 to 15 mass%, further more preferably 2.5 to 13 mass%, and particularly preferably 3 to 11 mass%.

### [4] Modified Starch

The modified starches used in the present invention can be obtained by using corn, potato, sweet potato, wheat, rice, glutinous rice, tapioca, sago palm and like starches as raw materials, and subjecting these starches to a chemical treatment that can be roughly classified into decomposition treatment and addition treatment.

These raw starch materials may be used singly or in a combination of two or more.

Preferable examples of raw starch material include corn and tapioca. Examples of known kinds of corn include dent corn (Zea mays Linn. var. indentata Sturt), flint corn (Zea mays Linn. var. indurata Sturt), soft corn (Zea mays Linn. var. amylacea Sturt), sweet corn (Zea mays Linn. var. saccharata Sturt), popcorn (Zea mays Linn. var. everta Sturt) and glutinous corn (waxy corn, Zea mays Linn. var. ceratina Sturt).

There is no particular limitation to the type of corn used in the present invention and any type of corn can be used as a raw starch material. Preferably the starch is derived from glutinous corn, i.e., waxy corn (hereafter simply referred to as "waxy corn" in this specification).

Examples of modified starches usable in the present invention include starches obtainable by processing the raw starch materials mentioned above. Specific examples include acetylated distarch adipate, acetylated oxidized starch, acetylated distarch phosphate, oxidized starch, hydroxypropyl starch, hydroxypropyl distarch phosphate, carboxymethyl starch, starch acetate, starch octenyl succinate, monostarch phosphate, distarch phosphate, phosphated distarch phosphate and the like. Among these, hydroxypropyl starch, hydroxypropyl distarch phosphate and starch octenyl succinate are preferable and starch octenyl succinate is particularly preferable. These modified starches may be used alone or as a mixture of two or more kinds.

The amount of the modified starch in the composition of the present invention is, per 100 parts by mass of curcumin, preferably in a range of 0.5 to 100 parts by mass, more preferably in a range of 1 to 80 parts by mass, further preferably in a range of 1 to 60 parts by mass, further more preferably in a range of 2 to 50 parts by mass, particularly preferably in a range of 3 to 40 parts by mass, more particularly preferably in a range of 3 to 35 parts by mass, in a range of 3 to 30 parts by mass, or in a range of 3 to 25 parts by mass, most preferably in a range of 4 to 15 parts by mass.

Further, the amount of the modified starch in the composition of the present invention is, per 100 parts by mass of gum arabic, preferably in a range of 0.5 to 300 parts by mass, more preferably in a range of 1 to 200 parts by mass, further preferably in a range of 3 to 100 parts by mass, further more preferably in a range of 3 to 60 parts by mass, particularly preferably in a range of 3 to 50 parts by mass, more particularly preferably in a range of 4 to 40 parts by mass, in a range of 5 to 35 parts by mass, or in a range of 6 to 20 parts by mass, and most preferably 8 to 15 parts by mass.

Further, the amount of the modified starch in the composition of the present invention is preferably in a range of 0.05 to 30 mass%, more preferably 0.1 to 20 mass%, further preferably 0.2 to 15 mass%, and further more preferably 0.3 to 11 mass%.

### [5] Water

The amount of water in the composition of the present invention is preferably in a range of 50 to 5000 parts by mass, more preferably 300 to 4000 parts by mass, further preferably 350 to 3500 parts by mass, further more preferably 400 to 30000 parts by mass, particularly preferably 500 to 1500 parts by mass, and more particularly preferably 500 to 1000 parts by mass, per 100 parts by mass of gum arabic.

As is evident to a person skilled in the art, the water in the composition of the present invention may form an aqueous medium. That is, for example, a pH adjuster described later, or the like may be dissolved in water. Further, any water generally recognized as water in the field of food products, including pure water, ion exchanged water, tap water, and the like, may be used.

### [6] Polyhydric Alcohol

The composition of the present invention preferably further comprises polyhydric alcohol.

Polyhydric alcohol may function as a preservative.

Examples of polyhydric alcohol used in the present invention include propylene glycol, glycerol, and a combination thereof.

Glycerol (glycerin) is preferably used as polyhydric alcohol in the present invention.

The amount of polyhydric alcohol in the composition of the present invention is preferably in a range of 10 to 1000 parts by mass, more preferably 100 to 800 parts by mass, further preferably 150 to 700 parts by mass, further more preferably 200 to 600 parts by mass, particularly preferably 300 to 550 parts by mass, and more particularly preferably 300 to 500 parts by mass, per 100 parts by mass of curcumin.

The amount of polyhydric alcohol in the composition of the present invention is preferably in a range of 100 to 2500 parts by mass, more preferably 300 to 2000 parts by mass, further preferably 400 to 1800 parts by mass, further more preferably 500 to 1500 parts by mass, particularly preferably 500 to 1300 parts by mass, per 100 parts by mass of gum arabic.

The amount of polyhydric alcohol in the composition of the present invention is preferably in a range of 100 to 20000 parts by mass, more preferably 500 to 18000 parts by mass, further preferably 1000 to 15000 parts by mass, further more preferably 1500 to 13000 parts by mass, and particularly preferably 2000 to 13000 parts by mass, per 100 parts by mass of modified starch.

The amount of polyhydric alcohol in the composition of the present invention is preferably in a range of 10 to 90 mass%, more preferably 20 to 80 mass%, further preferably 30 to 70 mass%, and further more preferably 35 to 60 mass%.

According to the research by the present inventors, contacting curcumin and gum arabic with polyhydric alcohol tends to generate aggregation, coagulation, or precipitation. In particular, when curcumin is pulverized in the presence of gum arabic and polyhydric alcohol, aggregation, coagulation, or precipitation is likely to occur.

However, such aggregation, coagulation or precipitation is suppressed in the composition of the present invention.

### [7] Other Components

Insofar as the effects of the present invention are not impaired, the curcumin composition of the present invention may further comprises additives, such as thickening polysaccharides, flavors, pigments, antioxidants, shelf-life improvers, preservatives (e.g., sodium benzoate), sugars, and the like. The use of such additives can impart variation in the properties of the curcumin composition itself, or variation in the sensory properties of the target to which the curcumin composition is added, such as taste, aroma, and/or texture, as well as various other properties thereof including storage stability, handling ease, and the like.

The total amount of the other components (i.e., components other than curcumin, gum arabic, modified starch, water, and polyhydric alcohol) in the composition of the present invention is, for example, 0.05 to 10 mass%.

### [8] Storage Property of Curcumin Composition

In the composition of the present invention, the generation of granular matter (the granular matter is typically conglomeration), which is problematic in terms of quality maintenance, is highly suppressed even when the composition is allowed to stand at a temperature of preferably 60°C for 3 days (preferably 7 days at 60°C). Such storage of the composition may start immediately after the production, or not immediately after the production.

Specifically, in microscopic observation, the number of granular matter having a major axis of 2 µm or more in randomly collected samples (n=5) is preferably less than 6 per field of view on average, more preferably less than 5 per field of view on average, more preferably less than 3 per field of view on average, and further more preferably less than 1 per field of view on average.

The diameter of the circle of the field of view (the sample surface observed by a microscope) is 0.3625 mm. A microscope with 40x objective lens and 15x eyepiece lens (field number: 22) is used.

### [9] Method for Producing Curcumin Composition

The composition of the present invention may be produced by a method comprising mixing curcumin, gum arabic, and water in the presence of modified starch.

The mixing may be performed by a suitable conventional method, using suitable conditions and apparatus.

The method for mixing curcumin, gum arabic, water, and optional polyhydric alcohol is not particularly limited as long as mixing can be performed. Examples of the mixing method include a method of stirring the components using a conventional stirrer.

When the composition of the present invention comprises curcumin, gum arabic, water, and polyhydric alcohol, the method for producing the composition preferably comprises mixing curcumin, gum arabic, water, and polyhydric alcohol in the presence of modified starch.

With this method, the aggregation of curcumin or a curcumin composition is suppressed even under conditions in which the aggregation of curcumin is accelerated, i.e., conditions in which curcumin and polyhydric alcohol coexist.

Specifically, for example, a method of adding modified starch, curcumin, gum arabic, water, and polyvalent alcohol to a container, and subsequently mixing and stirring them with a propeller or the like, thereby preparing an aqueous suspension containing these components may be used. The temperature in the stirring and mixing step is not particularly limited, and may be set in a range of 1 to 100°C. The duration of the stirring and mixing is also not particularly limited as it depends on the production scale, and may be set in a range of, for example, 1 to 60 minutes. The stirring speed is also not particularly limited as it depends on the production scale, and may be set in a range of, for example, 1 to 3000 RPM.

The aqueous suspension may be subsequently subjected to a pulverization treatment (treatment to obtain fine particles). Physical pulverization methods are preferably used as the pulverization treatment (treatment to obtain fine particles). An example of a physical pulverization method includes a treatment using a wet pulverizer. Specific examples of wet pulverizers include the Ultra visco mill (product name: Aimex) and Dyno-Mill (Dyno-Mill KDL (product name), Willy A. Bachofen AG). Other wet pulverizers, such as a sand mill (with beads), a star mill (Ashizawa Finetech Ltd.) and a Co-ball mill (product name: FrymaKoruma), can also be used.

The mixing and the pulverization treatment may be performed at the same time.

The mixing may also serve as a pulverization treatment. The mixing and the pulverization treatment may be performed at the same time using, for example, preferably Dyno-Mill.

To suppress the dissolution of curcumin, the mixing and the pulverization treatment may be performed at a pH of less than 9.

The aqueous suspension or the pulverized matter thereof may further be subjected to a homogenization treatment thereafter as required so as to evenly mix the respective components including curcumin fine particles. The homogenization method is not particularly limited as long as it can homogeneously disperse the curcumin and other components, and can be conducted using an emulsification and dispersion apparatus such as a nanomizer, microfluidizer and homogenizer, or an ultrasonic dispersion apparatus. The homogenization treatment loosens the aggregation of curcumin, thus further increasing its dispersibility to water and dispersion stability in water.

The pH of the composition of the present invention may be suitably adjusted depending on the target product to which the curcumin composition of the present invention is to be added (e.g., the target product to be colored (hereinafter referred to as a product to be colored)).

Preferably, the pH value is adjusted to be 8 or lower. Examples of pH adjusters to be used include phosphoric acid, sulfuric acid, hydrochloric acid and like inorganic acids; citric acid, lactic acid, malic acid and like organic acids; and salts thereof. These can be used singly, or in a combination of two or more. These pH adjusters may be used as appropriate depending on the type of the product to which the pH adjuster is added, and the targeted pH.

The composition of the present invention preferably has a pH in the range of 2 to 8.

A curcumin composition in a powder form (hereafter referred to as a curcumin powder composition) can be prepared, if necessary, by drying and powderizing the curcumin liquid composition of the present invention. The curcumin powder composition thus obtained is advantageous in that it is useful to prepare a product that contains curcumin with an extremely high concentration; it can be used to produce a dry product, such as a food or a tablet using a dry method; it is also highly preservable without requiring the addition of a preservative, etc. The dryer used for drying and powderization is not particularly limited, and examples thereof include a spray dryer, a slurry dryer and like spray dryers; a freeze dryer; etc.

By further subjecting the curcumin powder composition to granulation or subsequent tableting as necessary, the curcumin composition can be formed into granules (curcumin granular composition) or tablets (curcumin tablet composition). Such granules and tablets can be produced by adding additives known in the related fields (e.g., excipient, binder, lubricant, disintegrant, etc.) as necessary, according to a conventional preparation technique. In particular, a curcumin composition having a granular form is advantageous in that it exhibits high solubility and promptly dissolves when it is added to an aqueous product such as a beverage or a cosmetic lotion.

### [10] Use of Curcumin Composition

### (1) Use as a Food Additive or Additive

The curcumin composition of the present invention can be used as a food additive or an additive, more specifically as a flavoring agent or a coloring agent for various food products (including general food products (which also include health foods) and supplements such as dietary supplements), and cosmetics.

The foods may be in any form of liquid, semi solid, or solid, and specific examples thereof are as listed below.

Beverages (e.g., carbonated beverages, fruit beverages (including fruit juice, fruit juice-containing soft drinks, fruit juice-containing carbonated beverages, fruit pulp-containing beverages), vegetable juice, fruit and vegetable juice, low-alcoholic beverages, coffee beverages, powder beverages, sport beverages, supplement beverages, and tea (black tea, green tea, blend tea, etc.) beverages); desserts (e.g., custard puddings, milk puddings, fruit juice-containing puddings, jelly, Bavarian cream, etc.); frozen desserts (e.g., ice cream, milk ice cream, fruit juice-containing ice cream, soft ice cream, ice candies, sorbets, etc.); gum (e.g., chewing gum, bubble gum, etc.); chocolates (e.g., marble chocolate and like coating chocolates, strawberry chocolate, blueberry chocolate, melon chocolate, etc.); candies (e.g., hard candies (including bon-bons, butter balls, marbles, etc.)), soft candies (including caramel, nougat, gummy candy, marshmallows, etc.), drops, toffee, etc.); other confections (e.g., baked confections such as hard biscuits, cookies, okaki (rice crackers), senbei (rice crackers), etc.); soups (e.g., consomme soups, potage soups, pumpkin soups, etc.); tsukemono (Japanese pickles, e.g., asa-zuke, shoyu-zuke, shio-zuke, miso-zuke, kasu-zuke, koji-zuke, nuka-zuke, su-zuke, karashi-zuke, moromi-zuke, ume-zuke, fukujin-zuke, shiba-zuke, shoga-zuke, umezu-zuke, etc.); jams (e.g., strawberry jam, blueberry jam, marmalade, apple jam, apricot jam, etc.); milk products (e.g., milk beverages, lactic fermented milk drinks), yogurt, cheese, etc.); oil or fat-containing food products (e.g., butter, margarine, etc.); processed grain foods (e.g., breads, noodles, pasta, etc.); processed fish or animal food products (e.g., ham, sausage, kamaboko, chikuwa, etc.); seasonings (e.g., miso, tare (Japanese style sauces), sauces, bottled lemon juice, vinegar, mayonnaise, salad dressings, curry roux, etc.); cooked food products (e.g., tamago-yaki (Japanese omelets), omelets, curry, stew, hamburger patties, croquette, soups, okonomi-yaki (pan cakes with vegetables, meat or seafood), gyoza (fried or boiled dumplings), fruit jam, etc.).

Among these, beverages, jams, tsukemono, and liquid seasonings belonging to the sauce category are preferable, and beverages are particularly preferable. In the case of dietary supplements and like supplements, syrups, liquids and solutions, drinkable preparations, tablets, pills, powders, granules, and capsules are preferable.

When the curcumin composition of the present invention is used as a food additive or additive (coloring agent or flavoring agent), the product to be colored or flavored (e.g., foods, beverages, and cosmetics) can be produced by adding the curcumin composition of the present invention as a coloring agent or a flavoring agent in any step of producing the target product. Such a product can be produced according to a conventional method except that the aforementioned step is added.

In this case, the amount of the curcumin composition is not particularly limited as long as it serves the desired purpose. When coloring is the goal, specifically, the curcumin composition of the present invention may be added in such an amount that its proportion relative to the final product becomes 0.01 mass% at minimum.

Here, an example of known curcumin (a conventional curcumin composition) is a liquid, solubilized curcumin composition that can be obtained by extracting curcumin powder by hydrous ethanol using the crude curcumin and production method disclosed in the List of Existing Food Additives (Appendix 1 for Labeling and Specifications under the Food Sanitation Law in Notice No. 56, published May 23, 1996, by the Director-General of the Environmental Health Bureau, Ministry of Health and Welfare).

### (2) Use as a Functional Component

When the bioactive function of curcumin is targeted, the curcumin composition of the present invention itself can be used as supplements such as dietary supplements.

In this case, the curcumin composition of the present invention can be formed into "orally administered curcumin preparations", i.e., orally administrable preparations, such as hard capsules, soft capsules, tablets, granules, powders, fine granules, pills, troches, syrups, liquids and solutions, drinkable preparations, etc.

In this case, the dosage of the curcumin composition of the present invention (an orally administered curcumin preparation) depends on the consumer's age, body weight and condition, dosage form, duration of treatment and the like. According to the Technical Report from WHO, the ADI (Acceptable Daily Intake) of curcumin is 0 to 3 mg/kg of body weight/day, and the NOAEL (no-observed-adverse-effect level) is 250 to 320 mg/kg of body weight/day (WHO Technical Report Series: page 33). Therefore, the curcumin composition can be administered at one time or divided into several times within this range.

### [11] Pigment Preparation

As understandable from the above explanation regarding the composition of the present invention, the composition of the present invention may be a pigment preparation (or a coloring agent) in one embodiment of the present invention.

The present invention also provides products (e.g., food, pharmaceutical products, cosmetics) colored with the pigment preparation (or a coloring agent).

Preferable examples of the products encompasses food products colored with the composition of the present invention.

More specifically, for example, sugar-coated candies can be produced by coating candies with a sugar-coating syrup prepared by using the pigment preparation (or a coloring agent) of the present invention. As understandable from the present invention, the sugar-coating syrup and the sugar-coated candies are also embodiments of the composition of the present invention.

The method for applying the pigment preparation (or a coloring agent) of the present invention to these products and amount thereof may be selected or determined according to, for example, the products to be colored, the purpose of coloring, and the like, based on common technical knowledge.

### [12] Aggregation Inhibitor

The present invention provides an aggregation inhibitor for curcumin or curcumin compositions.

The aggregation inhibitor contains gum arabic and modified starch.

The modified starch may be an active ingredient of the aggregation inhibitor.

In the present invention, "suppression" of aggregation encompasses "prevention" of aggregation.

### [13] Dispersant

The present invention also provides a dispersant for curcumin.

The dispersant contains gum arabic and modified starch. The dispersant promotes the dispersion of curcumin in water.

The dispersant can enhance the retention of the curcumin dispersion state in water.

More specifically, the dispersant of the present invention may be an aggregation inhibitor for curcumin.

In the present invention, "suppression" of aggregation encompasses "prevention" of aggregation.

The dispersion of curcumin in water does not necessarily require the use of the dispersant for curcumin of the present invention. The aggregation inhibitor for curcumin of the present invention may be used after the curcumin dispersion in water is achieved.

As is evident to a person skilled in the art from the disclosure of the specification of the present invention, the present invention also provides a curcumin dispersion method, a curcumin aggregation inhibition method, and the like. The details thereof can be easily understood from the descriptions of the specification.

### Examples

The present invention is explained in detail below with reference to Examples; however, the present invention is not limited to the Examples.

Each symbol and abbreviation in the Examples stands for the following.

In the tables of the following Examples, numerals indicating formulations are parts by mass unless otherwise specified.

### Materials

In all Examples and Comparative Examples below, the following materials were used.
Turmeric Pigment: Curcumin powder No. 3705: San-Ei Gen F.F.I., Inc., crystalline form, curcumin content = 90%
Modified gum arabic: SUPER GUM (San-Ei Gen F.F.I., Inc.) Modified starch (octenyl succinic acid starch sodium): Purity gum BE (produced by Nippon Scientific Co., Ltd.)
Modified starch (hydroxypropyl starch): NATIONAL 7 (produced by Nippon Scientific Co., Ltd.)
Modified starch (hydroxypropyl distarch phosphate): NATIONAL FRIGEX (produced by Nippon Scientific Co., Ltd.)
Sucrose Fatty Acid Ester: DK ester SS (Dai-Ichi Kogyo Seiyaku Co., Ltd.)
Lecithin: HABO HHSL (Compass Foods Pte Ltd)

### Method for Producing a Preparation

Each preparation in the Examples and Comparative Examples below was produced by subjecting mixed solutions having respective formulations to a pulverization treatment using a wet pulverizer (Dyno-Mill KDL (product name), Willy A. Bachofen AG (WAB)), followed by processing with a homogenizer (high-pressure homogenizer 15MR-8TA, produced by APV GAULIN) at 20 MPa at room temperature.

### Evaluation

### 1. Pulverization Suitability

The pulverization suitability upon production of a preparation in the Examples and Comparative Examples strongly relates to the viscosity or fluidity of the pulverized preparation.

More specifically, for example, when the viscosity of the preparation significantly increases or when the fluidity of the preparation significantly decreases after the pulverization, pulverization is no longer possible. Therefore, the pulverization suitability of a preparation was determined based on the following discharge ratio (%) used as the index thereof.

### ▪ Determination of Discharge Ratio (%) and Judgement of Pulverization Suitability

The discharge ratio (%) as an index of viscosity or fluidity was determined as follows.

25 g of a sample was placed in a screw tube No. 6 (diameter 3 cm × height 6.5 cm), and adjusted in temperature to 50°C.

The tube was inclined at 120°, a sample mass (g) discharged in 5 seconds was measured, and the discharge ratio (%) was calculated.

The pulverization suitability was determined to be poor when the discharge ratio was less than 50%; and
The pulverization suitability was determined to be good when the discharge ratio was 50% or more.

The variation of the discharge ratio in the above method was very small. Table 1 shows the test result examples wherein n=3 (Comparative Examples 1 and 3, and Example 3 shown later).

**Table 1**

| | Discharge Amount (g) | Discharge Ratio (%) | Average Discharge Ratio (%) |
|---|---|---|---|
| Comparative Example 1 | 0.00 | 0.00 | 0 |
| | 0.00 | 0.00 | |
| | 0.00 | 0.00 | |
| Comparative Example 3 | 0.00 | 0.00 | 0 |
| | 0.00 | 0.00 | |
| | 0.00 | 0.00 | |
| Example 10 | 22.83 | 91.32 | 90 |
| | 22.44 | 89.76 | |
| | 22.29 | 89.16 | |

### 2. Storage Stability

The storage stability of each preparation of the Examples and Comparative Examples was evaluated by visual inspection by observing the appearance when the produced preparation was allowed to stand for a predetermined time at a predetermined temperature. The evaluation was performed in four scales using the following criteria. In the beginning of the storage stability test, granular matter was not observed.

### ▪ Evaluation Criteria for Storage Stability of Preparation

Poor (×): The number of granular matter having a maximum length of 2 µm or more among randomly collected samples (n=5) is not less than 6 per field of view on average, in microscope observation after the sample was allowed to stand for 5 months at 5°C, or for 3 days at 60°C (accelerated test).

Acceptable (Δ) : The number of granular matter having a maximum length of 2 µm or more among randomly collected samples (n=5) is less than 6 per field of view on average and not less than 3 per field of view on average, in microscope observation after the sample was allowed to stand for 5 months at 5°C, or for 3 days at 60°C (accelerated test).

Good (○): The number of granular matter having a maximum length of 2 µm or more among randomly collected samples (n=5) is less than 3 per field of view on average and not less than 1 per field of view on average, in microscope observation after the sample was allowed to stand for 5 months at 5°C, or for 3 days at 60°C (accelerated test) .

Excellent (⊚): The number of granular matter having a maximum length of 2 µm or more among randomly collected samples (n=5) is less than 1 per field of view on average, in microscope observation after the sample was allowed to stand for 5 months at 5°C, or for 3 days (accelerated test) at 60°C.

### Production Example 1 (Comparative Example: Preparation (containing gum arabic, and free of modified starch)

Preparations of the respective Comparative Examples containing modified gum arabic and free of modified starch were produced using a Dyno-Mill. These preparations had the formulations shown in the table below.

Table 2 shows pulverization suitability, median diameter of the particles obtained by pulverization, and storage stability of the preparation.

In the formulation containing glycerol (Comparative Example 3), the fluidity of the liquid composition disappeared by the problematic viscosity increase; further, pulverization using a Dyno-Mill was difficult. Therefore, the storage stability of the preparation was not tested.

The increase in viscosity, which becomes problematic during pulverization in the manufacture of a preparation was not observed in the formulations free of glycerol (Comparative Examples 4 and 5), and their pulverization suitability were all evaluated as "good." However, all preparations had inferior storage stability.

**Table 2**

| | Comparative Example 3 | Comparative Example 4 | Comparative Example 5 |
|---|---|---|---|
| Turmeric Pigment | 10.60 | 10.60 | 10.60 |
| Modified Gum Arabic | 6.50 | 4.00 | 5.30 |
| Sodium Benzoate | - | 0.10 | 0.10 |
| Citric Acid | - | 0.33 | 0.33 |
| Glycerol | 45.70 | - | - |
| Ion Exchanged Water | 37.20 | 84.97 | 83.67 |
| Total | 100.00 | 100.00 | 100.00 |
| Pulverization Suitability | Poor | Good | Good |
| Median Diameter (µm) | - | 0.214 | 0.255 |
| Preparation Storage Stability | - | Poor | Poor |

Preparations of the respective Comparative Examples containing unmodified gum arabic and free of modified starch were produced using a Dyno-Mill. These preparations had the formulations shown in the table below.

Table 3 shows pulverization suitability, median diameter of the particles obtained by pulverization, and storage stability of the preparation.

Upon the pulverization, aggregation occurred, and the fluidity of the liquid composition disappeared due to problematic viscosity; further, pulverization using a Dyno-Mill was difficult. Therefore, the median diameter of the preparation was not measured and the storage stability was not tested.

**Table 3**

| | Comparative Example 1 | Comparative Example 2 |
|---|---|---|
| Turmeric Pigment | 10.70 | 10.70 |
| Unmodified Gum Arabic | 11.80 | 9.80 |
| Citric Acid | 0.20 | 0.30 |
| Glycerol | 42.60 | 43.65 |
| Ion Exchanged Water | 34.70 | 35.55 |
| Total | 100.00 | 100.00 |
| pH | 3.92 | 3.86 |
| Pulverization Suitability | Poor | Poor |
| Median Diameter (µm) | - | - |
| Preparation Storage Stability | - | - |

### Remarks: The curcumin crystal was pulverized using a Dyno-Mill.

### Production Example 2 (Comparative Example: a preparation containing modified starch and free of gum arabic)

The preparation of Comparative Example 14 containing modified starch and free of gum arabic having the formulation shown in Table 4 was produced.

Table 4 shows pulverization suitability, median diameter of the particles obtained by pulverization, and storage stability of the preparation.

The increase in viscosity, which becomes problematic during pulverization in the manufacture of preparations, was not observed, and the pulverization suitability was evaluated as "good." However, the preparation had inferior storage stability. When the preparation was allowed to stand for five months at 5°C, the preparation itself was solidified. Further, when the precipitation was allowed to stand for five months at room temperature, precipitation was observed in the bottom of the storage container.

**Table 4**

| | Comparative Example 14 |
|---|---|
| Turmeric Pigment | 11.00 |
| Modified Starch | 11.60 |
| Sodium Benzoate | 0.10 |
| Trisodiun Citrate | 0.30 |
| Ion Exchanged Water | 77.00 |
| Total | 100.00 |
| pH | 4.70 |
| Pulverization Suitability | Good |
| Median Diameter (µm) | 0.191 |
| Preparation Storage Stability | Poor |

### Production Example 3 (Example: a preparation containing gum arabic and modified starch)

The preparations of the Examples containing modified gum arabic and modified starch having the formulations shown in Tables 5 and 6 were produced using Dyno-Mill.

Tables 5 and 6 show pulverization suitability, median diameter of the particles obtained by pulverization, and storage stability of the preparations.

The increase in viscosity, which becomes problematic during pulverization in the manufacture of preparations, was not observed in all preparations, and their pulverization suitability were all evaluated as "good." Further, all preparations had good storage stability.

The results showed that desirable pulverization suitability and preparation storage stability can be ensured when the ratio of the modified gum arabic to the modified starch is such that the modified starch falls within a range of 6.15 to 31.78 parts by mass, per 100 parts by mass of the modified gum arabic.

**Table 5**

| | Example 1 | Example 2 | Example 3 | Example 4 | Example 5 |
|---|---|---|---|---|---|
| Turmeric Pigment | 10.600 | 10.600 | 10.600 | 10.600 | 10.600 |
| Modified Gum Arabic | 6.500 | 6.500 | 5.300 | 4.000 | 4.000 |
| Modified Starch | 2.000 | 0.400 | 0.670 | 0.500 | 0.660 |
| Sodium Benzoate | - | - | - | - | - |
| Citric Acid | - | - | - | - | - |
| Glycerol | 44.400 | 45.500 | 52.400 | 46.670 | 46.400 |
| Ion Exchanged Water | 36.500 | 37.000 | 31.030 | 38.230 | 38.340 |
| Total | 100.000 | 100.000 | 100.000 | 100.000 | 100.000 |
| pH | 4.45 | 4.48 | 4.61 | 4.50 | 4.53 |
| Pulverization Suitability | Good | Good | Good | Good | Good |
| Median Diameter(µm) | 0.386 | 0.349 | 0.227 | 0.347 | 0.339 |
| Preparation Storage Stability | Excellent | Excellent | Excellent | Excellent | Excellent |

**Table 6**

| | Example 6 | Example 7 | Example 8 | Example 9 | Example 10 |
|---|---|---|---|---|---|
| Turmeric Pigment | 10.600 | 10.600 | 10.600 | 10.600 | 10.600 |
| Modified Gum Arabic | 9.000 | 9.000 | 10.400 | 10.400 | 10.700 |
| Modified Starch | 0.770 | 0.630 | 0.880 | 0.720 | 3.400 |
| Sodium Benzoate | 0.100 | 0.100 | 0.100 | 0.100 | 0.100 |
| Citric Acid | 0.300 | 0.300 | 0.300 | 0.300 | 0.300 |
| Glycerol | - | - | - | - | - |
| Ion Exchanged Water | 79.230 | 79.370 | 77.720 | 77.880 | 74.900 |
| Total | 100.000 | 100.000 | 100.000 | 100.000 | 100.000 |
| pH | 3.45 | 3.43 | 3.42 | 3.61 | 3.40 |
| Pulverization Suitability | Good | Good | Good | Good | Good |
| Median Diameter (µm) | 0.362 | 0.281 | 0.342 | 0.312 | 0.263 |
| Preparation Storage Stability | Excellent | Excellent | Excellent | Excellent | Excellent |

### Production Example 4 (Example: a preparation containing unmodified gum arabic and modified starch)

The preparations of the Examples containing unmodified gum arabic and modified starch having the formulations shown in Table 7 were produced using Dyno-Mill.

Table 7 shows pulverization suitability, median diameter of the particles obtained by pulverization, and storage stability of the preparation. The preparations of these Examples all had a pH in the range of 3.3 to 3.6.

The increase in viscosity, which becomes problematic during pulverization in the manufacture of preparations, was not observed in all preparations, and their pulverization suitability were all evaluated as "good." Further, all preparations had acceptable storage stability.

The results showed that the problematic viscosity increase was not observed when the addition amount of the unmodified gum Arabic was 15 mass% or less; that is, good pulverization suitability was confirmed.

**Table 7**

| | Example 11 | Example 12 | Example 13 | Example 14-1 | Example 14-2 | Example 15 | Example 16 |
|---|---|---|---|---|---|---|---|
| Turmeric Pigment | 10.60 | 11.00 | 11.00 | 16.60 | 14.40 | 13.30 | 11.00 |
| Unmodified Arabic | 12.70 | 6.00 | 3.00 | 3.00 | 3.00 | 3.00 | 3.00 |
| Modified Starch | 1.60 | 3.60 | 3.60 | 5.40 | 4.70 | 4.30 | 3.60 |
| Sodium Benzoate | 0.10 | 0.10 | 0.10 | 0.10 | 0.10 | 0.10 | - |
| Citric Acid | 0.23 | - | - | - | - | - | - |
| Glycerol | - | - | - | - | - | - | 40.10 |
| Ion Exchanged Water | 74.77 | 79.30 | 82.30 | 74.90 | 77.80 | 79.30 | 42.30 |
| Total | 100.00 | 100.00 | 100.00 | 100.00 | 100.00 | 100.00 | 100.00 |
| Pulverization Suitability | Good | Good | Good | Good | Good | Good | Good |
| Median Diameter(µm) | 0.39 | 0.20 | 0.21 | 0.22 | 0.21 | 0.22 | 0.21 |
| Preparation Storage Stability | Acceptable | Acceptable | Acceptable | Acceptable | Acceptable | Acceptable | Acceptable |

### Production Example 5 (Comparative Example: a preparation containing sucrose fatty acid ester and modified starch)

The preparations containing sucrose fatty acid ester and modified starch having the formulations shown in Table 8 were produced using Dyno-Mill.

The preparations were all properly obtained, and the pulverization suitability was evaluated as good. However, all preparations had inferior storage stability. More specifically, unlike the preparation containing gum arabic, the storage stability was inferior even in the combined use of modified starch.

**Table 8**

| Composition | Comparative Example 15 | Comparative Example 16 | Comparative Example 17 |
|---|---|---|---|
| Turmeric Pigment | 11.00 | 11.00 | 11.00 |
| Modified Starch | 3.60 | 3.60 | 3.60 |
| Sucrose Fatty Acid Ester | 15.00 | 30.00 | 15.00 |
| Lecithin | - | - | 3.00 |
| Glycerol | 35.60 | 21.20 | 33.80 |
| Ion Exchanged Water | 34.80 | 34.20 | 33.60 |
| Total | 100.00 | 100.00 | 100.00 |
| Pulverization Suitability | Poor | Good | Good |
| Median Diameter (µm) | - | 0.24 | 0.18 |
| Preparation Storage Stability | Poor | Poor | Poor |

### Example 17: Sugar-Coated Candy

A sugar-coated candy syrup was prepared according to the formulation shown in Table 9 using the curcumin dispersion preparation obtained in Example 5.

**Table 9**

| | |
|---|---|
| Sugar | 170 |
| Gum Arabic | 8.5 |
| Water | 84 |
| Total | 262.5 g |

The raw materials of the syrup having the above formulation were mixed and boiled down until the Brix became 72 degrees.

1 g of the curcumin dispersion preparation of Example 5 was added to 29 g of the obtained boiled liquid, thereby preparing sugar-coated candy syrup.

60 g of spherical hard candies having a diameter of 1.5 cm were coated with 1 g of the syrup prepared above, and the coated candies were gently dried using a sugar-coating pan, thereby preparing sugar-coated candies.

The prepared sugar-coated candies were confirmed to be evenly colored with no unevenness and were confirmed to have a bright vivid yellow tone both in observation of the surface of a single candy and observation of a plurality of candies.

Further, sugar-coated candies were prepared in the same manner as that described above after the curcumin dispersion preparation of Example 5 was stored for a week at 60°C; as a result, evenly-colored bright vivid yellow sugar-coated candies were obtained as above.

The color tone was the same as that of the sugar-coated candies prepared by using a curcumin dispersion preparation before storing for a week at 60°C.

### Production Example 6 (Example: a preparation containing modified gum arabic and various modified starches)

The preparations of the Examples having the formulations shown in Table 10 were produced in the same manner as that of Production Example 3, except that various modified starches were used.

Table 10 shows pulverization suitability, median diameter of the particles obtained by pulverization, and storage stability of the preparation.

The increase in viscosity, which becomes problematic during pulverization in the manufacture of preparations, was not observed in all preparations, and their pulverization suitability was all evaluated as "good". Further, all preparations had excellent or acceptable storage stability.

**Table 10**

| | Example 18 | Example 19 | Example 20 | Example 21 |
|---|---|---|---|---|
| Turmeric Pigment | 10.60 | 10.60 | 10.60 | 10.60 |
| Modified Gum Arabic | 9.00 | 9.00 | 9.00 | 4.00 |
| Modified Starch (Octenyl Succinic Acid Starch Sodium) | 1.00 | - | - | - |
| Modified Starch (Hydroxypropyl Starch) | - | 1.00 | - | 0.50 |
| Modified Starch (Hydroxypropyl Distarch | - | - | 1.00 | - |
| Sodium Benzoate | 0.10 | 0.10 | 0.10 | - |
| Citric Acid | 0.30 | 0.30 | 0.30 | - |
| Glycerol | - | - | - | 46.67 |
| Ion Exchanged Water | 79.00 | 79.00 | 79.00 | 38.23 |
| Total | 100.00 | 100.00 | 100.00 | 100.00 |
| pH | 3.55 | 3.53 | 3.53 | 4.76 |
| Pulverization Suitability | Good | Good | Good | Good |
| Median Diameter (µm) | 0.310 | 0.379 | 0.410 | 0.284 |
| Preparation Storage Stability | Excellent | Good | Good | Acceptable |

## Claims

1. A composition comprising curcumin, gum arabic, modified starch, and water, and having a median diameter of 1 µm or less.

2. The composition according to claim 1, wherein the composition is a liquid composition.

3. The composition according to claim 1 or 2, wherein the composition contains curcumin as a turmeric pigment.

4. The composition according to any one of claims 1 to 3, wherein the amount of curcumin is in a range of 0.5 to 30 mass%.

5. The composition according to any one of claims 1 to 4, wherein the gum arabic is modified gum arabic.

6. The composition according to any one of claims 1 to 5, wherein the amount of gum arabic is in a range of 10 to 250 parts by mass, per 100 parts by mass of curcumin.

7. The composition according to any one of claims 1 to 6, wherein the amount of modified starch is in a range of 0.5 to 100 parts by mass, per 100 parts by mass of curcumin.

8. The composition according to any one of claims 1 to 7, wherein the amount of modified starch is in a range of 0.5 to 300 parts by mass, per 100 parts by mass of gum arabic.

9. The composition according to any one of claims 1 to 8, wherein the composition further contains polyhydric alcohol.

10. The composition according to claim 9, wherein the polyhydric alcohol is glycerol.

11. The composition according to claim 9 or 10, wherein the amount of polyhydric alcohol is in a range of 10 to 1000 parts by mass, per 100 parts by mass of curcumin.

12. A method for producing the composition according to claims 1 to 11, comprising mixing curcumin, gum arabic, and water in the presence of modified starch.

13. The method for producing the composition according to claim 12, the method comprising mixing curcumin, gum arabic, water, and polyhydric alcohol in the presence of modified starch.

14. The method according to claim 12 or 13, the method comprising pulverizing the mixture obtained by the mixing.

15. The composition according to any one of claims 1 to 11, wherein the composition is a pigment preparation.

16. A food product colored with the composition according to claim 15.

17. A dispersant for curcumin, comprising gum arabic and modified starch.

18. The dispersant according to claim 17, wherein the dispersant is an aggregation inhibitor for curcumin.
